# NEUE EUROPÄISCHE PATENTSCHRIFT

(11) **EP 0 697 881 B2**
(45) Veröffentlichungstag und Bekanntmachung des Hinweises auf die Entscheidung über den Einspruch: **06.05.2009**
(45) Hinweis auf die Patenterteilung: 12.06.2002
(21) Anmeldenummer: 94915073.4
(22) Anmeldetag: 20.04.1994
(51) Int. Cl.: A61K 38/00, A61K 47/14, A61K 9/20, A61K 9/48

(54) **NEUE PHARMAZEUTISCHE ZUBEREITUNGEN ZUR ORALEN VERABREICHUNG ENTHALTEND CYCLOSPORIN**
NEW CYCLOSPORINE-CONTAINING PHARMACEUTICAL COMPOSITIONS FOR ORAL ADMINISTRATION
NOUVELLES COMPOSITIONS PHARMACEUTIQUES POUR ADMINISTRATION ORALE CONTENANT DE LA CYCLOSPORINE

(30) Priorität: 20.04.1993 DE 4312728; 08.04.1994 DE 4412201
(43) Veröffentlichungstag der Anmeldung: 28.02.1996
(73) Patentinhaber: NOVARTIS AG, 4056 Basel (CH); Novartis-Erfindungen Verwaltungsgesellschaft m.b.H., 1235 Wien (AT)
(72) Erfinder: FLECK, Monika, D-88471 Laupheim (DE); NEUER, Klaus, D-88477 Schwendi (DE); WALCH, Hatto, D-88471 Laupheim (DE)
(86) Internationale Anmeldenummer: PCT/EP1994/001228
(87) Internationale Veröffentlichungsnummer: WO 1994/023733

(56) Entgegenhaltungen:
- EP-A- 0 365 044
- CH-A- 641 356
- FR-A- 2 642 650
- GB-A- 2 222 770
- GB-A- 2 228 198
- GB-A- 2 230 440
- US-A- 4 990 337
- 'Rote Liste 1992' , BUNDESVERBAND DER PHARMAZEUTISCHE INDUSTRIE E.V. , FRANKFURT DE "Sandimmun kapseln" siehe Seite 50078
- RITSCHEL W.A. ET AL METH AND FIND EXP CLIN PHARMACOL Bd. 11, Nr. 4, 1989, Seiten 281 - 287

## Beschreibung

Die Erfindung betrifft neue pharmazeutische Zubereitungen für die orale Verabreichung enthaltend Cyclosporin als Wirkstoff.

Cyclosporine sind eine Klasse von Peptiden, die insbesondere als Immunsuppressiva verwendet werden. Darüber hinaus ist bekannt, daß Cyclosporine eine entzündungshemmende und antiparasitäre Wirkung aufweisen. Der Einsatz der Cyclosporine ist daher nicht nur auf Immunsuppressiva beschränkt, sondern betrifft alle entzündlichen Erkrankungen einschließlich verschiedener Autoimmunerkrankungen sowie weitere entzündliche Zustände, insbesondere entzündliche Zustände in denen Autoimmunprozesse eine Rolle spielen. Zu den vorgenannten entzündlichen Zuständen zählen insbesondere auch die arthritischen Erkrankungen wie z. B. rheumatoide Arthritis sowie rheumatische Erkrankungen. Als antiparasitäres Mittel können Cyclosporine z. B. zur Behandlung von Protozoeninfektionen wie z. B. Malaria eingesetzt werden.

Cyclosporine sind stark hydrophobe Substanzen, mit der Folge, daß es schwierig ist, sie einfach zu pharmazeutischen Zubereitungen zu verarbeiten, die weiterhin eine ausreichende Bioverfügbarkeit gewährleisten. Der letztere Aspekt ist insbesondere im Hinblick darauf wichtig, weil die Cyclosporine nephrotoxische Nebenwirkungen von wesentlicher Bedeutung besitzen. Bislang vorgeschlagene cyclosporinhaltige pharmazeutische Zubereitungen basieren auf der Verwendung eines Alkohols und/oder Ölen oder ähnlichen Vehikeln in Verbindung mit einem oberflächenaktiven Mittel. Derartige Zubereitungen sind z. B. aus der DE-OS 29 07 460 bekannt. Die Verwendung derartiger flüssiger Zusammensetzungen ist jedoch von einer Reihe von Nachteilen und Schwierigkeiten begleitet. Die Verwendung von Ölen oder damit vergleichbaren Trägerstoffen auf Ölbasis führt insbesondere bei längerer Anwendungszeit im Rahmen einer Langzeittherapie zu Geschmacksbeeinträchtigungen. Da zur Lösung des Wirkstoffs ein hoher Alkoholanteil erforderlich ist, bedingt dies, daß zudem dem Patienten permanent Alkohol zugeführt wird und bei Verdampfen des Alkohols im Rahmen eines längeren Gebrauchs fällt der Wirkstoff aus. Auch der Versuch, derartige Zubereitungen in form von Weichgelacinekapseln anzubieten, führte aufgrund des damit verbundenen erhöhten Aufwandes zu keiner zufriedenstellenden Lösung.

Die DE-05 40 03 844 schlägt ein Zubereitungssystem vor, das neben dem Wirkstoff einen Fettsäuresaccharidmonoester und ein Verdünnungsmittel oder Trägerstoff enthält, mit dem es möglich sein soll, feste, halbfeste und flüssige Zubereitungen mit einem Gehalt an Cyclosporin in ausreichend hoher Konzentration zur Verfügung zu stellen, daß damit eine bequeme orale Verabereichung möglich ist und eine verbesserte Wirksamkeit zum Beispiel in bezug auf die Bioverfügbarkeitseigenschaften erreicht wird. Demnach enthalten diese Verabreichungsformen neben dem Wirkstoff mindestens zwei weitere Komponenten.

CH-A-641 356 betrifft Cyclosporin-enthaltende Zusammensetzungen, die mindestens eine der folgenden Komponenten als Träger enthalten: ein Transesterifikationsprodukt eines Triglycerids mit einem Polyalkylenglykol, ein gesättigtes Fettsäuretriglycerid und ein Mono- oder Diglycerid.

EP-A-0 365 044 betrifft neue Verwendungen von (Nva)²-Cyclosporin. Die offenbarten Cyclosporin-Zusammensetzungen enthalten einen Träger mit einem HLB-Wert unter 10.

Die Anmelderin hat nun überraschenderweise ein Zubereitungssystem für die orale Verabreichung gefunden, mit dem es möglich ist, eine cyclosporinhaltige pharmazeutische Zubereitung für die orale Verabreichung zur Verfügung zu stellen, die neben dem Wirkstoff Cyclosporin lediglich eine Trägerkomponente enthält. Diese Komponente ist ein Alkylenpolyether oder -ester oder eine beliebige Mischung davon, worin das Trägersystem einen HLB-Wert von mindestens 10 aufweisen muß. Die erfindungsgemäßen Zubereitungen ergeben eine Bioverfügbarkeit des Wirkstoffs, der mindestens mit den besten bekannten cyclosporinhaltigen Zubereitungen vergleichbar ist.

Die erfindungsgemäßen pharmazeutischen Zubereitungen sind bei vergleichbar guter Bioverfügbarkeit wirtschaftlicher herzustellen, vermeiden geschmacksbeeinträchtigende Zusätze sowie den nachteiligen Alkoholgehalt und führen darüber hinaus zu einer verbesserten Patientencompliance in dem Sinne, daß die einzunehmende Zubereitungsform bei gleicher Wirkstoffkonzentration im Vergleich zu bekannten Zubereitungen im Gesamtgewicht reduziert ist.

Die Erfindung betrifft demnach pharmazeutische Zubereitungen zur oralen Verabreichung, die Cyclosporin als Wirkstoff enthalten und wie folgt zusammengesetzt sind:
a) ein Cyclosporin als Wirkstoff,
b) ein Alkylenpolyether oder -ester als Trägerstoff oder eine beliebige Mischung davon, wobei der HLB-Wert mindestens 10 ist.

Fakultativ können die erfindungsgemäßen Zubereitungen als weitere Komponente (c) ein Alkylenpolyol, ein Alkylenglykol, ein Polyalkylenglykol, einen C₂₋₅-Alkyldi- oder Teilether eines niedermolekularen Mono- oder Polyoxyalkandiols mit 2 bis 15 Kohlenstoffatomen enthalten und/oder ein pflanzliches Öl oder dessen hydriertes oder hydrolysiertes Produkt enthalten.

Darüber hinaus können die erfindungsgemäßen Zubereitungen weitere bekannte, übliche und pharmazeutisch annehmbare Zusatzstoffe (d) wie sie auf dem Gebiet der Herstellung von oralen Zubereitungsformen bekannt sind, enthalten.

Die erfindungsgemäßen Zubereitungen enthalten in Gewichtsanteilen pro Gewichtsteil Wirkstoff 1 bis 50 Gewichtsteile von (b) bzw. 0,5 bis 20 Gewichtsteile von (c), bevorzugt pro 1 Teil Wirkstoff 5 bis 10 Gewichtsteile (b) bzw. 1 bis 10 Gewichtsteile (c) und insbesondere pro 1 Gewichtsteil Wirkstoff 5 Gewichtsteile (b) bzw. 1 Gewichtsteil (c).

Bei der Komponente (b) handelt es sich geeigneterweise um C₃ bis C₅ Alkylentriol-, insbesondere Glycerin, -ether oder -ester. Dazu zählen auch z.B. Umesterungsprodukte der Alkylencriolester mit anderen Mono-, Di- oder Polyolen sowie jene Substanzen, die in der DE-OS 40 03 844 unter dem Abschnitt "Komponente C⁵" beschrieben sind. Besonders vorteilhaft sind gesättigte polyglykolisierte Glyceride, die einen HLB-Wert von mindestens 10 aufweisen. Vorzugsweise werden die unter der Markenbezeichnung Gelucire (die Bezeichnung Gelucire ist ein Warenzeichen der Fa. Gattefossé bekannten gesättigten polyglykolisierten Glyceride verwendet und insbesondere die Gelucire^{R} 35/10, 44/14, 42/12, 50/13, 53/10 und beliebige Mischungen davon, wobei der HLB-Wert der verwendeten Trägerkomponente mindestens 10 ist.

Die fakultative Komponente (c) umfaßt z.B. Diether oder Teilether von niedermolekularen (C₂₋₁₂) Mono- oder Polyoxyalkandiolen wie sie in der DE-OS 39 30 928 im Abschnitt betreffend die Komponente 1.1. beschrieben sind. Die fakultative Komponente (c) umfaßt weiterhin C₃₋₅-Alkylenpolyole, C₂₋₄-Alkylenglykole, Poly-(C₂₋₄-alkylen)-glykole, und pflanzliche Öle sowie deren Hydrierungs- und/oder Hydrolyseprodukte, wie z. B. Ricinusöl, Olivenöl, Palmöl, Kokosöl, Maisöl, Sesamöl. Die Komponente (c) kann als Einzelsubstanz oder in beliebigen Mischungen enthalten sein. Bevorzugte Beispiele für die Komponente (c) sind Glycerin, Propylenglykol und Polyalkylenglykole mit einem Molekulargewicht bis insbesondere 600, Transcutol und Ricinusöl und dessen hydrierte und hydrolysierte Produkte.

Bei den weiteren verwendbaren Zusatzstoffen handelt es sich um auf dem Gebiet der oralen Darreichungsformen übliche pharmazeutisch annehmbare Zusatzstoffe. Beispiele dafür sind die Freisetzung steuernde Stoffe, Verdickungsmittel, Konservierungsmittel, Stabilisatoren, Geschmacksstoffe, Bindemittel, Gleitmittel und dergleichen. Der Anteil dieser Zusatzstoffe kann bis 50% der Gesamtzusammensetzung ausmachen, beträgt jedoch bevorzugt nicht mehr als 25 und insbesondere nicht mehr als 10% der Gesamtzusammensetzung.

Zur Verwendung in den erfindungsgemäßen Zubereitungen sind alle bekannten natürlichen und synthetischen Cyclosporine einschließlich ihre Analoge und Derivate geeignet. Beispiele für derartige Cyclosporine finden sich z. B. in der DE-OS 40 03 844 und DE-OS 40 05 190. Bevorzugt wird Cyclosporin A verwendet.

Zu den oralen Darreichungsformen zählen beispielsweise Flüssigkeiten, Granulate und feste Formen wie Tabletten und Kapseln, die nach den dem Fachmann bekannten und üblichen Verfahren hergestellt werden können.

Die erfindungsgemäßen oralen Darreichungsformen liegen üblicherweise als Einheitsdosisform vor und enthalten etwa 20 bis 200 mg, bevorzugt 50 oder 100 mg Wirkstoff, pro Einheitsdosis.

Die folgenden Beispiele dienen der weiteren Erläuterung der Erfindung.

### Beispiele

| 1. | Bestandteile | Anteil (mg) |
|---|---|---|
| | Cyclosporin A | 50,0 |
| | Gelucir 53/10 | 300,0 |
| | | |
| | **Gesamt** | **3̅5̅0̅,̅0̅** |
| | | |
| 2. | Cyclosporin A | 50,0 |
| | Gelucir 44/14 | 250,0 |
| | Propylenglykol | 50,0 |
| | | |
| | **Gesamt** | **3̅5̅0̅,̅0̅** |
| | | |
| 3. | Cyclosporin A | 50,0 |
| | Gelucir 50/13 | 250,0 |
| | Transcutol | 75,0 |
| | | |
| | **Gesamt** | **3̅7̅5̅,̅0̅** |
| | | |
| 4. | Cyclosporin A | 50,0 |
| | Gelucir 44/14 | 250,0 |
| | | |
| | **Gesamt** | **3̅0̅0̅,̅0̅** |
| | | |
| 5. | Cyclosporin A | 50,0 |
| | Gelucir 50/13 | 250,0 |
| | Propylenglykol | 50,0 |
| | | |
| | **Gesamt** | **3̅5̅0̅,̅0̅** |
| 6. | Cyclosporin A | 50,0 |
| | Gelucir 35/10 | 250,0 |
| | Propylenglykol | 25,0 |
| | | |
| | **Gesamt** | **3̅2̅5̅,̅0̅** |
| | | |
| 7. | Cyclosporin A | 50,0 |
| | Gelucir 53/10, 42/12 | 275,0 |
| | Transcutol | 50,0 |
| | | |
| | **Gesamt** | **3̅7̅5̅,̅0̅** |
| | | |
| 8. | Cyclosporin A | 50,0 |
| | Gelucir 42/12 | 300,0 |
| | Glycerin | 25,0 |
| | | |
| | **Gesamt** | **3̅7̅5̅,̅0̅** |
| | | |
| 9. | Cyclosporin A | 50,0 |
| | Gelucir 50/13 | 250,0 |
| | Ricinusöl | 75,0 |
| | | |
| | **Gesamt** | 3̅7̅5̅,̅0̅ |

Herstellung: Die Zusammensetzungen der Beispiele 1 bis 9 werden dadurch hergestellt, daß die Komponente (b) unter Erwärmen, bevorzugt auf mindestens 60°C, geschmolzen und darin unter Rühren der Wirkstoff (a) gelöst wird. Gegebenenfalls wird der Schmelze die fakultative Komponente (c) zugesetzt.

Die erhaltenen Zubereitungen werden anschließend beispielsweise in flüssiger Form in Hartgelatinekapseln gewünschter Größe in den gewünschten Konzentrationen abgefüllt. Die Zusammensetzungen können auch in bekannter Weise zu Tabletten weiterverarbeitet werden. Dazu werden die Schmelzen wie oben beschrieben hergestellt. Die flüssigen Schmelzen werden ausgegossen und nach dem Erstarren mit einer Siebmaschine zerkleinert. Die so hergestellten Granulate werden mit den üblichen Hilfsstoffen wie Gleit- und Schmiermitteln, Sprengmitteln, Füllstoffen, Geschmackskorrigenzien usw. vermischt. Die fertigen Mischungen werden zu Tabletten mit dem gewünschten Gehalt an Cyclosporin verpreßt. Die Tabletten können ebenfalls mit einer Schutzhülle überzogen werden.

### Bioverfügbarkeit:

Untersuchunger. zur Bioverfügbarkeit der erfindungsgemäßen Zusammensetzungen am Hund.

Für die BV-Untersuchungen wurde eine Gruppe von sechs Beagle-Hunden eingesetzt. Die Applikation der Prüfpräparate erfolgte oral mittels Schlundsonde am nüchternen Tier. Den Tieren wird zu definierten Zeitpunkten Blut aus der Vena saphena entnommen und in entsprechenden Kunststoffröhrchen mit EDTA-Zusatz gesammelt. Die Blutproben werden bis zur Bestimmung bei -18°C gelagert. Die Bestimmung des Cyclosporins erfolgt im Vollblut mittels Fluoreszenz-Polarisations-Immunoassay (FPIA).
Die Flächen unter den Kurven (AUC), bei denen die Blutarzneistoffkonzentration gegen die Zeit aufgetragen wird, wurden nach der Trapezregel berechnet. Die durchschnittlichen AUC-Werte von erfindungsgemäßen Zusammensetzungen sind in der folgenden Tabelle im Vergleich zu den im Handel befindlichen Präparaten Cyclosporin Trinklösung und Cyclosporin Kapseln (Sandimmun^{R}), die in gleicher Weise, bei gleicher Dosierung mit den gleichen Hunden ermittelt wurden, dargestellt.

| Beispiele | AUC (0-12 h) ng/ml |
|---|---|
| Beispiel 2 | 9035 ± 2134 |
| Beispiel 3 | 7859 ± 1512 |
| Beispiel 4 | 7552 ± 1194 |
| Beispiel 5 | 8228 ± 857 |
| Cyclosporin Trinklösung | 7980 ± 1320 |
| Cyclosporin Kapseln | 8098 ± 1504 |

Wie die vorstehenden Versuche zur Bioverfügbarkeit zeigen, ist es mit den erfindungsgemäßen pharmazeutischen Zubereitungen möglich den Wirkstoff Cyclosporin in einer solchen Form oral zur Verfügung zu stellen, daß dessen Bioverfügbarkeit mindestens den bislang bekannten Zubereitungen entspricht.

## Patentansprüche

1. Pharmazeutische Zubereitung zur oralen Verabreichung bestehend aus
(a) einem Cyclosporin als Wirkstoff und
(b) einem Alkylenpolyether or -ester entweder alleine oder in jeder beliebigen Mischung als Trägerstoff, wobei der HLB-Wert der eingesetzten Komponente (b) mindestens 10 ist.

2. Zubereitung nach Anspruch 1, in der die Komponente (b) aus gesättigten polyglykolisierten Glyceriden ausgewählt ist.

3. Zubereitung nach Anspruch 2, in der die Komponente (b) aus den Geluciren Gelucir^{R} 35/10, 44/14, 42/12, 50/13, 53/10 und beliebigen Mischungen davon ausgewählt ist.

4. Pharmazeutische Zubereitung zur oralen Verabreichung enthaltend
(a) ein Cyclosporin als Wirkstoff,
(b1) ein gesättigtes polyglykolisiertes Glycerid ausgewählt aus den Geluciren Gelucir^{R} 35/10, 44/14, 42/12, 50/13, 53/10 oder einer beliebigen Mischung wobei der HLB-Wert der eingesetzten Komponente (b1) mindestens 10 ist, und
(c) ein Alkylenpolyol, ein Alkylenglykol, ein Polyalkylenglykol, ein Alkyldi- oder Teilether eines niedermolekularen Mono- oder Polyoxyalkandiols mit 2 bis 15 Kohlenstoffatomen und/oder ein pflanzliches Öl oder dessen hydriertes oder hydrolysiertes Produkt entweder alleine oder in beliebigen Mischungen.

5. Zubereitung nach Anspruch 4, in der die jeweiligen Komponenten (a), (b) bzw (c) in folgenden Gewichtsverhältnissen vorhanden sind: 1:1-50:0.5-20, bevorzugt 1:5-10:1-10, insbesondere 1:5:1.

6. Zubereitung nach Anspruch 4, in der die Komponente (c) aus Glycerin, Propylenglykol, PEG mit MG bis etwa 600, Transcutol und Rhizinusöl ausgewählt ist.

7. Zubereitung nach einem der vorhergehenden Ansprüche in Form einer Hardgelatinekapsel.

8. Zubereitung nach einem der Ansprüche 1 bis 6 in Form einer Tablette.

9. Zubereitung nach einem der vorhergehenden Ansprüche, **dadurch gekennzeichnet, dass** die Wirkstoffkonzentration 20 bis 200 mg pro Dosiseinheit beträgt.

10. Zubereitung nach einem der vorhergehenden Ansprüche, **dadurch gekennzeichnet, dass** die Wirkstoffkonzentration 50 to 100 mg pro Dosiseinheit beträgt.

11. Zubereitung nach einem der vorhergehenden Ansprüche wobei das Cyclosporin Cyclosporin A ist.

## Claims

1. Pharmaceutical preparation for oral administration, containing
(a) a cyclosporin as active ingredient, and
(b) an alkylene polyether or alkylene polyester either alone or in any mixture as carrier, whereby the HLB of component (b) is at least 10.

2. Preparation according to claim 1, in which component (b) is chosen from saturated polyglycolised glycerides.

3. Preparation according to claim 2, in which component (b) is chosen from the Gelucires Gelucir^{®} 35/10, 44/14, 42/12, 50/13, 53/10 and any mixtures thereof.

4. Pharmaceutical preparation for oral administration, containing
(a) a cyclosporin as active ingredient,
(b1) a saturated polyglycolised glyceride chosen from the Gelucires Gelucir^{®} 35/10, 44/14, 42/12, 50/13, 53/10 and any mixture thereof, whereby the HLB of component (b1) is at least 10, and
(c) an alkylene polyol, an alkylene glycol, a polyalkylene glycol, an alkyldiether or partial ether of a lower monooxyalkanediol or polyoxyalkanediol with 2 to 15 carbon atoms and/or a vegetable oil or its hydrated or hydrolysed product either alone or in any mixtures.

5. Preparation according to claim 4, in which the respective components (a), (b) and (c) are present in the following weight ratios: 1:1-50:0,5-20, preferably 1:5-10:1-10, especially 1:5:1.

6. Preparation according to claim 4, in which component (c) is chosen from glycerine, propylene glycol, PEG with MW up to approximately 600, transcutol and castor oil.

7. Preparation according to one of the preceding claims in the form of a hard gelatin capsule.

8. Preparation according to one of claims 1 to 6 in the form of a tablet.

9. Preparation according to one of the preceding claims, **characterized in that** the active ingredient concentration is 20 to 200 mg per dose unit.

10. Preparation according to one of the preceding claims, **characterized in that** the active ingredient concentration is 50 to 100 mg per dose unit.

11. Preparation according to one of the preceding claims, in which the cyclosporin is cyclosporin A.

## Revendications

1. Préparation pharmaceutique pour administration orale, comprenant
(a) une cyclosporine en tant que principe actif, et
(b) un alkylènepolyéther ou un alkylènepolyester, seul ou selon un mélange quelconque, en tant qu'excipient, la valeur HLB du composant (b) utilisé étant d'au moins 10.

2. Préparation selon la revendication 1, dans laquelle le composant (b) est choisi parmi les glycérides polyglycolisés saturés.

3. Préparation selon la revendication 2, dans laquelle le composant (b) est choisi parmi les Gelucires Gelucire® 35/10, 44/14, 42/12, 50/13, 53/10 et les mélanges quelconques de ceux-ci.

4. Préparation pharmaceutique pour administration orale contenant
(a) une cyclosporine en tant que principe actif,
(b1) un glycéride polyglycolisé saturé choisi parmi les Gelucires Gelucire® 35/10, 44/14, 42/12, 50/13, 53/10 ou un mélange quelconque de ceux-ci, la valeur HLB du composant (b1) utilisé étant d'au moins 10, et
(c) un alkylènepolyol, un alkylèneglycol, un polyalkylèneglycol, un diéther ou éther partiel allylique d'un mono- ou polyoxyalcanediol de faible poids moléculaire ayant 2 à 15 atomes de carbone et/ou une huile végétale ou l'un de ses produits hydrogénés ou hydrolysés, à titre individuel ou selon des mélanges quelconques.

5. Préparation selon la revendication 4, dans laquelle les composants respectifs (a), (b) et (c) sont présents selon des rapports pondéraux suivants : 1:1-50:0,5-20, de préférence 1:5-10:1-10, en particulier 1:5:1.

6. Préparation selon la revendication 4, dans laquelle le composant (c) est choisi parmi le glycérol, le propylèneglycol, les PEG ayant un poids moléculaire allant jusqu'à environ 600, le Transcutol et l'huile de ricin.

7. Préparation selon l'une des revendications précédentes, sous forme d'une gélule en gélatine dure.

8. Préparation selon l'une des revendications 1 à 6, sous forme d'un comprimé.

9. Préparation selon l'une des revendications précédentes, **caractérisée en ce que** la concentration du principe actif est de 20 à 200 mg par dose unitaire.

10. Préparation selon l'une des revendications précédentes, **caractérisée en ce que** la concentration du principe actif est de 50 à 100 mg par dose unitaire.

11. Préparation selon l'une des revendications précédentes, dans laquelle la cyclosporine est la cyclosporine A.
